# EUROPEAN PATENT APPLICATION

(11) **EP 4 643 770 A1**
(43) Date of publication of application: **05.11.2025**
(21) Application number: 23912424.1
(22) Date of filing: 26.07.2023
(51) Int. Cl.: A61B 5/087, A61B 5/08, A61B 5/00

(54) **METHOD FOR MANUFACTURING BIOMATERIAL-BASED WEARABLE SENSOR**

(30) Priority: 29.12.2022 KR 20220189667
(71) Applicant: Chung Ang University Industry Academic Cooperation Foundation, Seoul 06974 (KR)
(72) Inventor: KIM, Sung Han, Seoul 07980 (KR); LEE, Chan Ui, Goyang-si Gyeonggi-do 10508 (KR); CHO, Hyeon Ho, Seoul 01752 (KR)
(74) Representative: Jell, Friedrich
(86) International application number: PCT/KR2023/010820
(87) International publication number: WO 2024/143737

(57) **Abstract**

The present invention relates to a method for manufacturing a biomaterial-based wearable sensor. The method for manufacturing a biomaterial-based wearable sensor according to the present invention is characterized by comprising: a) a first preparation step for preparing a base substrate in the form of a thin film; b) a second preparation step for placing an aqueous solution of graphene oxide and an aqueous silk fibroin solution in separate containers to prepare a material with which to coat the base substrate; c) a dip coating step for sequentially dip coating the base substrate with the graphene oxide aqueous solution and then the silk fibroin aqueous solution; and d) a drying step for drying the dip-coated base substrate by applying heat.

## Description

### Technical Field

The present invention relates to a biomaterial-based wearable sensor manufacturing method and, more specifically, to a biomaterial-based wearable sensor manufacturing method, capable of increasing durability against repeated use and an external load while having a characteristic of changing resistance according to temperature or humidity, and precisely manufacturing a biomaterial-based sensor at low cost.

### Background Art

Recently, services in the medical field have been expanding beyond the existing concept of "medical", which means diagnosis and treatment, to the concept of "healthcare", which means prevention and healthcare. In order to substantially introduce the concept of healthcare, it is necessary to develop a wearable sensor that is attached to the skin and can monitor body information in real time. Body information (respiration, etc.) acquired in real time through a wearable sensor in daily life is analyzed through big data and artificial intelligence-based technology, transmitted to a doctor, diagnosed, and the results are transmitted back to the user. In this conceptual diagram, the excellent mechanical properties of the wearable sensor are important to be usable in daily life. In addition, since it should be attached to the skin and used, it should be based on biomaterials that are bio-friendly, and it should be light and small to minimize inconvenience to users. In addition, a simple manufacturing method should be used to reduce production costs. However, the development of low-cost, compact wearable sensors with excellent mechanical properties based on biomaterials remains limited.

### -Prior Art Document

Korean Patent Publication No. 10-2006-0096386.

### Disclosure

### Technical Problem

The present invention has been devised to solve the above problems, and its object is to provide method for manufacturing a biomaterial-based wearable sensor that enhances the durability of sensors having a characteristic in which resistance is changed according to temperature or humidity, while enabling precisely manufacturing the sensor at a low cost by using biocompatible biomaterials.

### Technical Solution

In order to accomplish the above objects, the present invention provides a biomaterial-based wearable sensor manufacturing method, the method comprising: a) a first preparation step of preparing a thin film-shaped base substrate; b) a second preparation step, of preparing a material to be dip-coated on the base substrate by putting an aqueous graphene oxide solution and an aqueous silk fibroin solution in separate containers, respectively; c) a dip coating step of sequentially dip-coating the aqueous graphene oxide solution and the aqueous silk fibroin solution on the base substrate; and d) a drying step of drying the base substrate subjected to the dip coating by applying thermal reduction treatment.

The dip coating step may further include a first washing step of performing a washing process using a first deionized water after dip coating the graphene oxide aqueous solution on the base substrate and then dip coating the silk fibroin aqueous solution; and a second washing step of further performing a washing process using a second deionized water after dip coating the silk fibroin aqueous solution on the base substrate.

When the process from a) to d) is defined as one cycle, it is preferable to be configured to obtain a required thickness by repeatedly performing at least 2 cycles.

The dip coating step may include a container transfer operation of sequentially placing a container accommodating the graphene oxide aqueous solution, a container accommodating the first deionized water, a container accommodating the silk fibroin aqueous solution, and a container accommodating the second deionized water on a conveyor moving along a closed-loop track in order, and moving the container to a dip coater performing a dip coating process in a step manner.

The dip coating step may include: a sensing step of sensing whether each of the containers is positioned vertically below the dip coater; and an inflow step of allowing the base substrate held by the clamping member of the dip coater to immerse into the aqueous solution of the container for a predetermined period of time based on the sensing result.

The sensing step may be configured to identify the position of the container by sensing the load of the container using a load cell disposed below the deep coater of the conveyor.

When the conveyor is made of a light transmittable material, the light transmitting unit is coupled to the clamping member of the dip coater, a light receiving unit for performing a sensing operation by an interaction with the light transmitting unit is disposed below the conveyor corresponding to a vertically lower side of the light transmitting unit, and a light blocking film made of a light reflecting material is coupled to a lower surface of the container, the sensing step, when the container is not positioned vertically lower than the dip coater, may enable the movement of the conveyor by light reception between the light transmitting unit and the light receiving unit, and when the container is positioned vertically lower than the dip coater and thus light reception by the light blocking film is not performed, the movement of the conveyor may be stopped and a dip coating process may be performed.

The graphene oxide aqueous solution is preferably 0.3 wt %, and the silk fibroin aqueous solution is preferably 0.1 wt %, and the base substrate is preferably made of a wood pulp material.

### Advantageous Effects

The biomaterial-based wearable sensor manufacturing method according to the present invention having the configuration as described above is configured to sequentially coat an aqueous graphene oxide solution for implementing electrical properties and an aqueous solution of silk fibroin, which is an eco-friendly biomaterial, for improving mechanical properties (elasticity and durability), on an eco-friendly base substrate such as wood pulp by a dip coating method in which a coating process is relatively easy to implement, thereby deriving an effect of making it possible to manufacture a bio-friendly wearable sensor harmless to a human body at a low cost and deriving an effect of allowing a product to be used for a long time due to an effect of improving the mechanical properties of the silk fibroin even though the wearable sensor is attached to a human body to be repeatedly deformed and an external load is applied to wearable sensor from external conditions such as physical movement or environmental exposure.

### Brief Description of Drawings

FIG. 1 is a block diagram for explaining a configuration of a biomaterial-based wearable sensor manufacturing method according to an embodiment of the present invention.
FIG. 2 is a diagram for describing an implementation process of an embodiment of the present disclosure.
FIGS. 3 to 7 are graphs of experimental results for describing advantages of a sensor manufactured according to an embodiment of the present invention.
FIG. 8 is a view for describing an actual use example of a sensor manufactured according to an exemplary embodiment of the present invention.
FIGS. 9 to 11 are views for describing a device configuration and a manufacturing process according to another embodiment of the present invention.

### Best Mode

The following embodiments are detailed descriptions for helping the understanding of the present invention, and it will be natural that they do not limit the scope of the present invention. Therefore, an equivalent invention that performs the same function as the present invention will also fall within the scope of the present invention.

In the following description, the same identification symbols refer to the same configuration, and unnecessary redundant descriptions and descriptions of known technologies will be omitted. In addition, the following description of each embodiment of the present invention overlapping with the description of the background technology of the present invention will also be omitted.

Hereinafter, a biomaterial-based wearable sensor manufacturing method according to an embodiment of the present invention will be described in detail with reference to the accompanying drawings.

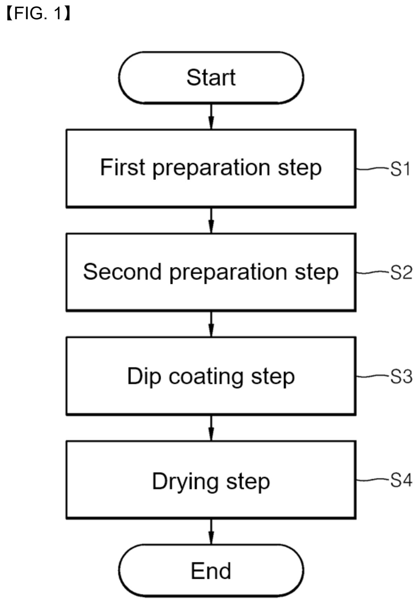

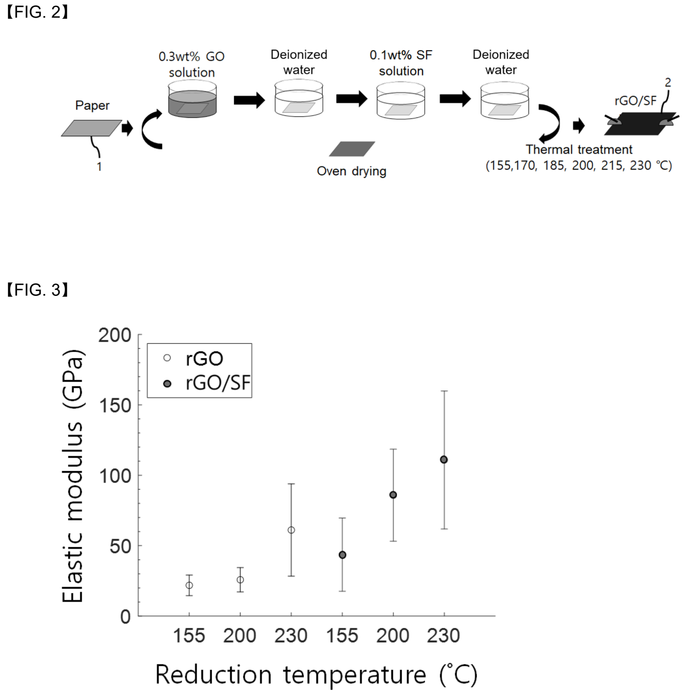

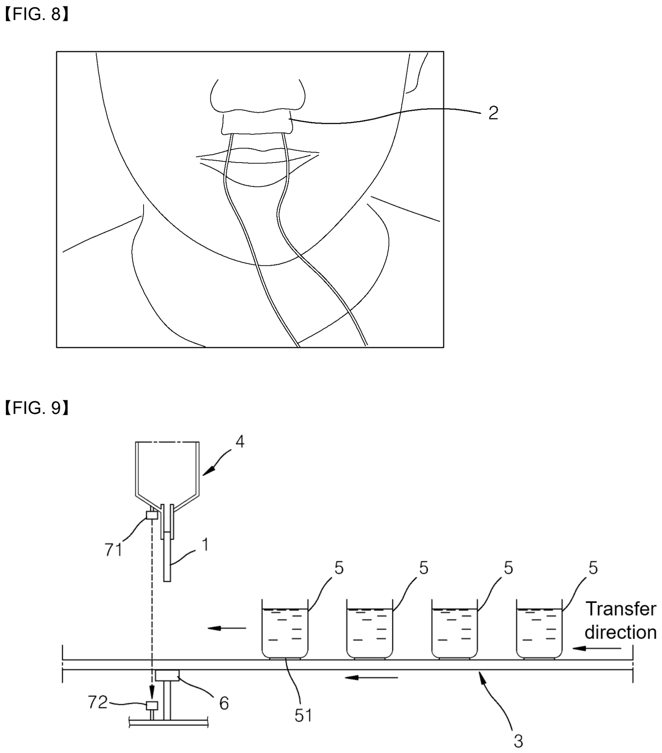

FIG. 1 is a block diagram for explaining a configuration of a biomaterial-based wearable sensor manufacturing method according to an embodiment of the present invention, FIG. 2 is a diagram for explaining an implementation process of an embodiment of the present invention, FIGS. 3 to 7 are graphs of experimental results for explaining advantages of a sensor manufactured according to an embodiment of the present invention, and FIG. 8 is a diagram for explaining an actual use example of a sensor manufactured according to an embodiment of the present invention.

As shown in FIGS. 1 and 2, the biomaterial-based wearable sensor manufacturing method according to an embodiment of the present invention is to manufacture a bio-friendly wearable sensor capable of measuring temperature and humidity on the basis of silk fibroin and wood pulp-based paper extracted from silkworm cocoons, and includes a first preparation step (S1), a second preparation step (S2), a dip coating step (S3), and a drying step (S4).

The first preparation step (S1) is a step of preparing a base substrate 1 having a thin film shape, and for example, a process of preparing a paper based on an eco-friendly material such as wood pulp is performed.

In the second preparation step S2, a process of preparing a material to be coated on the base substrate 1 by putting an aqueous solution of graphene oxide (GO) and an aqueous solution of silk fibroin (SF) in separate containers is performed. Here, in order to reduce manufacturing costs and implement a sensing function, it is preferable that the aqueous graphene oxide solution is 0.3 wt % and the aqueous solution of silk fibroin is 0.1 wt %.

Here, the graphene oxide aqueous solution was prepared by mixing graphene oxide with demineralized water (deionized water), and the silk fibroin aqueous solution was prepared by mixing silk fibroin with demineralized water. Graphene oxide (GO) is a material produced by attaching an oxygen functional group to graphene, and silk fibroin (SF) is a biomaterial extracted from cocoons.

The dip-coating step (S3) is to sequentially dip-coat the aqueous solution of graphene oxide and the aqueous solution of silk fibroin on the base substrate 1 by a well-known dip-coating method, and is organically combined with the above-mentioned eco-friendly material-based paper preparation step(S1), the coating material (graphene oxide and silk fibroin) preparation step(S2), and the drying step (S4) to be described later, thereby making it possible to manufacture a sensing product having excellent mechanical properties at low cost.

That is, as well shown in FIG. 3, as a result of an experiment with respect to a reduction temperature of a film having a structure in which the graphene oxide and the silk fibroin are sequentially stacked on the base substrate 1, it can be seen that the elastic modulus when the silk fibroin is present is increased by a maximum of 230% compared to the elastic modulus when the silk fibroin is not present, and thus the mechanical properties of the entire structure are improved due to the excellent bonding force between the silk fibroin and the graphene oxide.

In the drying step (S4), a process of drying the base substrate 1 that has undergone the dip coating by applying thermal reduction treatment is performed. For example, the dip-coated object may be manufactured by heating the dip-coated object at any one temperature of 155, 177, 185, 200, 215, and 230° C. for 3 hours. At this time, GO has an electrical conductivity as the oxygen functional group is eliminated, the defect disappears, and the graphene-like form is formed. The higher the heating to a higher temperature, the more defects disappear, making it close to graphene, obtaining excellent electrical conductivity.

The biomaterial-based wearable sensor manufacturing method according to an embodiment of the present invention having such a configuration is configured to sequentially coat the graphene oxide aqueous solution for implementing electrical properties and the eco-friendly silk fibroin aqueous solution for improving mechanical properties (elasticity and durability) on the eco-friendly material-based base substrate 1, such as wood pulp, by a dip coating method that is relatively easy to implement a coating process, thereby making it possible to manufacture a bio-friendly wearable sensor harmless to a human body at a low cost, and to expect the advantage of being applied to a human body and allowing a product to be used for a long time due to the effect of improving the mechanical properties of the silk fibroin even if repeated deformation and external load are applied to the wearable sensor.

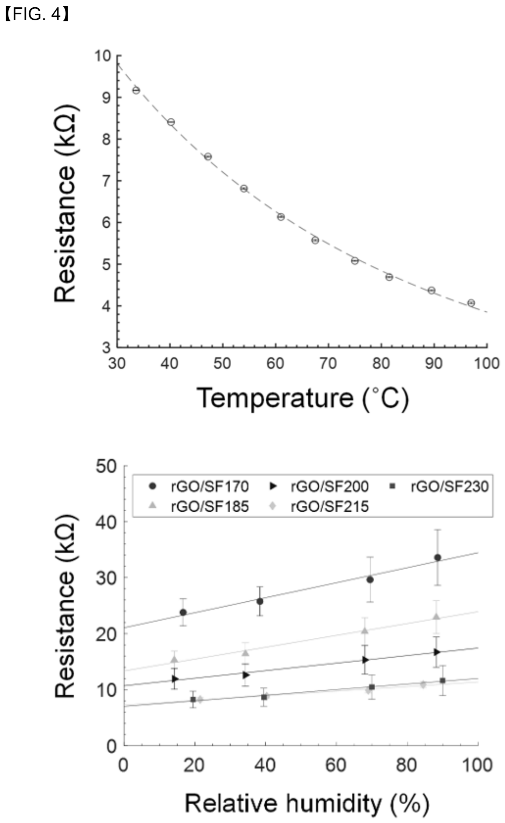

As well shown in FIG. 4, the sensor 2 manufactured through the manufacturing method according to the present embodiment described above may be used as a temperature/humidity sensor as resistance, which is one of electrical characteristics, is changed according to temperature and humidity through an experiment. The principle of measuring the temperature and humidity of the sensor 2 manufactured as described above is due to the fact that the oxygen functional group of the reduced graphene oxide/silk fibroin (rGO/SF) interacts with surrounding water molecules differently depending on the temperature and relative humidity to change the moisture content of the rGO/SF, resulting in a change in resistance due to different electrical characteristics.

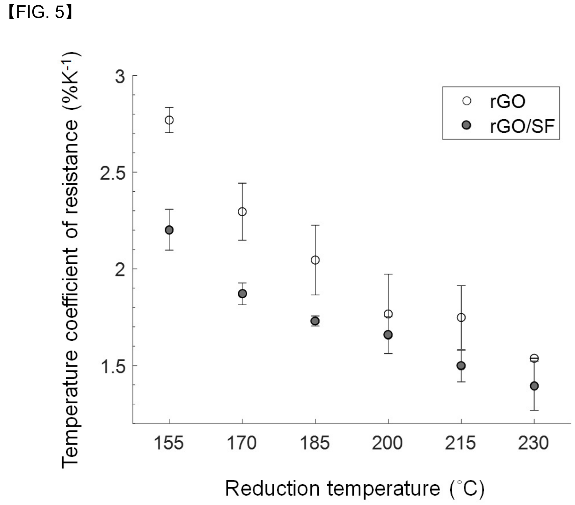

Further, as well shown in FIG. 5, the performance of the sensor varies according to the thermal reduction treatment conditions because the number of oxygen functional groups of the reduced graphene oxide (rGO) is affected by the thermal reduction treatment conditions. When the thermal reduction treatment temperature increases, the oxygen functional group of rGO is more dropped and the oxygen functional group is a medium that interacts with surrounding water molecules, so if the oxygen functional group decreases, the interaction with water molecules is weakened and the performance of the sensor changes accordingly.

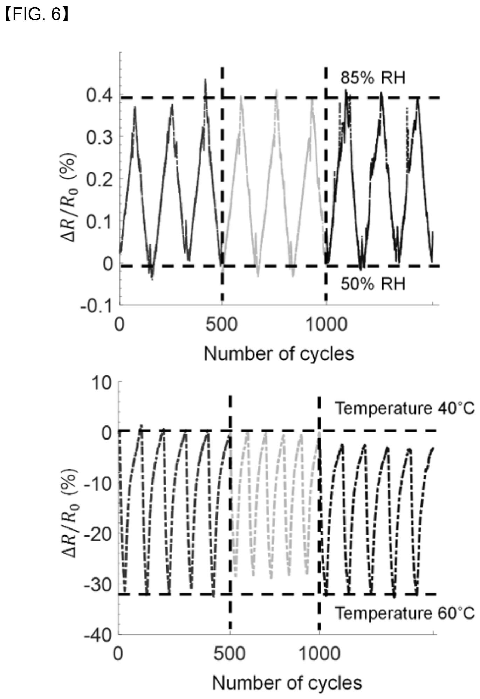

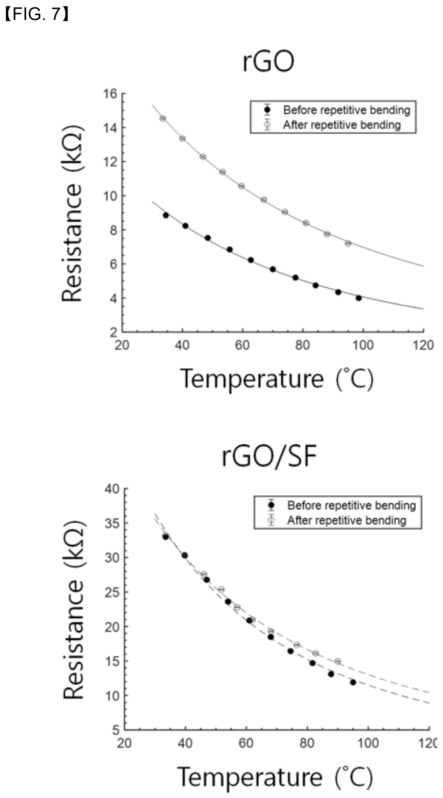

In addition, as shown in FIGS. 6 and 7, it was confirmed that the performance of the sensor was well implemented even when the relative humidity and temperature were repeatedly changed 1500 times and the load was 100 times. That is, as a result of an experiment on performance degradation of the sensor according to the repeated load, it was confirmed that when silk fibroin was not present, the electrical properties changed before and after the repeated load was applied due to low mechanical properties, and on the contrary, when silk fibroin was present, there was little change in electrical properties due to excellent mechanical properties before and after the repeated load was applied, so the robustness of the sensor was confirmed.

From these results, it can be seen that the wearable sensor manufactured by the present embodiment is suitable for use in real life. For example, as shown in FIG. 8, the wearable sensor 2 manufactured according to the present embodiment may be attached to a human body and utilized for monitoring a bio-signal, such as measuring a respiration rate.

That is, the expiratory of the respiration increases the temperature and the humidity of the philtrum. In addition, the inspiratory of the respiration decreases the temperature and the humidity of the philtrum. The sensor 2 manufactured according to the present embodiment may detect changes in humidity and temperature of the respiration to measure the respiration volume.

Meanwhile, the dip coating step S3 employed in the present embodiment further includes a first washing step and a second washing step for the precise manufacture of a sensor. In the first washing step, a desalting process using a first deionized water is performed after dip-coating the aqueous solution of graphene oxide on the base substrate 1 and before dip-coating the aqueous solution of silk fibroin on the base substrate 1, and in the second washing step, a washing process using a second deionized water is additionally performed after dip-coating the aqueous solution of silk fibroin on the base substrate 1, thereby removing excessively dip-coated graphene oxide and silk fibroin to manufacture a precise sensor.

The present embodiment having such a configuration has an advantage of obtaining a required thickness by repeating at least 2 cycles when the processes from the first preparation step S1 to the second preparation step S2, the dip coating step S3, and the drying step S4 described above are defined as 1 cycle.

Hereinafter, a biomaterial-based wearable sensor manufacturing method according to another embodiment of the present invention will be described in detail with reference to FIGS. 9 to 11.

FIGS. 9 to 11 are views for describing a device configuration and a manufacturing process according to another embodiment of the present invention.

The dip coating step employed in this embodiment includes a container transfer step, a sensing step, and an inflow step.

In the container transfer step, as shown in FIG. 9, a process of sequentially transferring a plurality of containers 5 containing a solution is performed. That is, a container in which the graphene oxide aqueous solution is accommodated, a container in which the first deionized water is accommodated, a container in which the silk fibroin aqueous solution is accommodated, and a container in which the second deionized water is accommodated are placed on a conveyor 3 moving along a closed-loop track in order, and the containers 5 are moved to a dip coater performing a dip coating process in a stepwise manner.

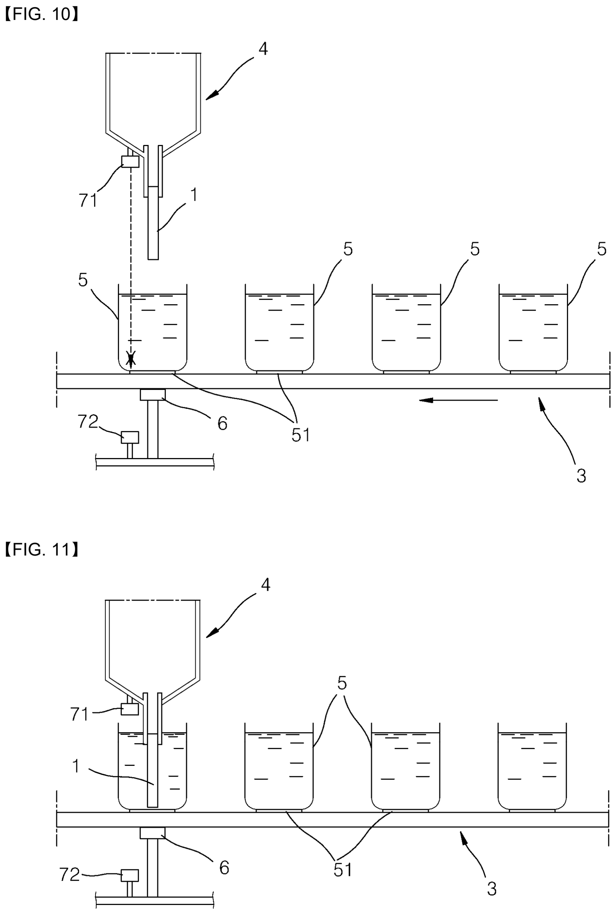

In the sensing step, as shown in FIG. 10, it serves to sense whether each container 5 is positioned below the deep coater vertical, and in the inflow step, as shown in FIG. 11, it serves to immerse the base substrate 1 held by the clamping member 4 of the deep coater into the aqueous solution of the container 5 for a predetermined period of time based on the sensing result.

The present embodiment having such a configuration has an advantage in that it is possible to further improve the efficiency of the sensor production by detecting whether each container 5 is disposed below the deep coater vertically after the containers 5 required for the sensor production are sequentially disposed on the conveyor 3, and automatically performing the deep coating process based on the detected signal.

The sensing step may be implemented by various configurations, but in the present embodiment, it is configured to identify the position of the container 5 by detecting the load of the container 5 using a load cell 6 disposed on the lower side of the deep coater of the conveyor 3.

Meanwhile, a sensing step for identifying the position of the container 5 may be implemented using the optical elements 71 and 72 separately from the load cell 6.

In order to implement the sensing step, it is preferable that the conveyor 3 is made of a light transmittable material, the light transmitting part 71 is coupled to the clamping member 4 of the dip coater, the light receiving part 72 for performing a sensing operation through interaction with the light transmitting part 71 is disposed below the conveyor 3 corresponding to a vertical lower portion of the light transmitting part 71, and a light blocking film 51 made of a light-reflectable material is coupled to a lower surface of the container 5, which is made of a transparent material.

In the sensing step, when the container 5 is not positioned vertically below the dip coater, the movement of the conveyor 3 is possible by the optical reception between the optical transmission unit 71 and the optical reception unit 72, and when the container 5 is positioned vertically below the dip coater and thus the optical reception by the light blocking film 51 is not performed, the movement of the conveyor 3 is stopped and a dip coating process is performed.

The present embodiment having such a configuration is configured such that a sensing step for automatically identifying the position of the container 5 is primarily implemented by the load cell 6 and is secondarily implemented through the optical elements 71 and 72, and thus, even when a load detection error phenomenon caused by the load cell 6 occurs due to a problem such as the looseness of the conveyor 3, the position of the container 5 can be identified through the optical elements 71 and 72, thereby enabling the construction of a more precise automation process.

Although various embodiments of the present invention have been described above, the present embodiment and the drawings attached to the present specification are merely clearly showing a part of the technical idea included in the present invention, and it will be obvious that all modified examples and specific embodiments that can be easily inferred by those skilled in the art within the scope of the technical idea included in the specification and drawings of the present invention are included in the scope of the present invention.

## Claims

1. A biomaterial-based wearable sensor manufacturing method, the method comprising: a) a first preparation step of preparing a thin film-shaped base substrate; b) a second preparation step of preparing a material to be coated on the base substrate by putting each of an aqueous graphene oxide solution and an aqueous silk fibroin solution in a separate container; c) a dip coating step of sequentially dip-coating the aqueous graphene oxide solution and the aqueous silk fibroin solution on the base substrate; and d) a drying step of drying the base substrate subjected to the dip coating by applying heat.

2. The biomaterial-based wearable sensor manufacturing method of claim 1, wherein the dip coating step further comprises: a first washing step of performing a washing process using a first deionized water after dip coating the graphene oxide aqueous solution on the base substrate and before dip coating the silk fibroin aqueous solution on the base substrate; and a second washing step of further performing a washing process using a second deionized water after dip coating the silk fibroin aqueous solution on the base substrate.

3. The biomaterial-based wearable sensor manufacturing method of claim 2, wherein when the processes a) to d) are defined as one cycle, at least two cycles are repeated to obtain a required thickness.

4. The biomaterial-based wearable sensor manufacturing method of claim 2, wherein the dip coating step comprises a container transfer step of sequentially placing a container in which the graphene oxide aqueous solution is accommodated, a container in which the first deionized water is accommodated, a container in which the silk fibroin aqueous solution is accommodated, and a container in which the second deionized water is accommodated on a conveyor that is moved along a closed-loop track, and moving the container to a dip coater side on which a dip coating process is performed.

5. The biomaterial-based wearable sensor manufacturing method of claim 4, wherein the dip coating step comprises: a sensing step of sensing whether each container is positioned vertically below a dip coater; and an inflow step of immersing a base substrate held by a clamping member of the dip coater into an aqueous solution of the container for a predetermined period of time based on the sensing result.

6. The biomaterial-based wearable sensor manufacturing method of claim 5, wherein the sensing step is configured to identify the position of the container by sensing a load of the container using a load cell disposed below the deep coater of the conveyor.

7. The biomaterial-based wearable sensor manufacturing method of claim 5, wherein when the conveyor is made of a light transmittable material, the clamping member of the dip coater is coupled with a light transmitting unit, a light receiving unit for performing a sensing operation by an interaction with the light transmitting unit is disposed below the conveyor corresponding to a vertically lower protion of the light transmitting unit, and a light shielding film made of a light-reflecting material is coupled to a lower surface of the container, the sensing step enables the movement of the conveyor by light reception between the light transmitting unit and the light receiving unit when the container is not positioned vertically below the dip coater, and stops the movement of the conveyor to perform a dip coating process when the container is positioned vertically below the dip coater and light reception by the light shielding film is not performed.

8. The biomaterial-based wearable sensor manufacturing method of claim 1, wherein the graphene oxide aqueous solution is 0.3 wt % and the silk fibroin aqueous solution is 0.1 wt %.

9. The biomaterial-based wearable sensor manufacturing method of claim 1, wherein the base substrate includes a wood pulp material.
